# EUROPEAN PATENT SPECIFICATION

(11) **EP 3 911 956 B1**
(45) Date of publication and mention of the grant of the patent: **12.07.2023**
(21) Application number: 21704596.2
(22) Date of filing: 18.01.2021
(51) Int. Cl.: G01N 33/569, G01N 33/543

(54) **METHOD FOR DETERMINING CORONAVIRUS**
VERFAHREN ZUR BESTIMMUNG VON CORONAVIRUS
PROCÉDÉ DE DÉTERMINATION DE CORONAVIRUS

(30) Priority: 06.04.2020 FI 20205357
(43) Date of publication of application: 24.11.2021
(73) Proprietor: Medicortex Finland OY, 20520 Turku (FI)
(72) Inventor: HAREL, Adrian, 20520 Turku (FI); KVIST, Mårten, 20520 Turku (FI); VÄLIMAA, Lasse, 20520 Turku (FI); UTZ, Begüm, 20520 Turku (FI); HAAVISTO, Oskar, 20520 Turku (FI); NURMI, Venla-Mari, 20520 Turku (FI)
(74) Representative: Hovinen, Jari Juhani
(86) International application number: PCT/FI2021/050026
(87) International publication number: WO 2021/205058

(56) References cited:
- WO-A1-91/06311
- WO-A1-2013/088367
- ADAM G ET AL: "Lectins as probes for the assay of rhabdovirus infections in plants", JOURNAL OF VIROLOGICAL METHODS, ELSEVIER BV, NL, vol. 17, no. 3-4, 1 September 1987 (1987-09-01), pages 263-275, XP023696254, ISSN: 0166-0934, DOI: 10.1016/0166-0934(87)90136-4 [retrieved on 1987-09-01]
- TORTORICI M ALEJANDRA ET AL: "Structural basis for human coronavirus attachment to sialic acid receptors", NAT. STRUCT. MOL. BIOL, NATURE PUBLISHING GROUP US, NEW YORK, vol. 26, no. 6, 1 June 2019 (2019-06-01), pages 481-489, XP037066010, ISSN: 1545-9993, DOI: 10.1038/S41594-019-0233-Y [retrieved on 2019-06-03]
- HU SHEN ET AL: "Lectin microarray", PROTEOMICS CLINICAL APPLICATIONS, vol. 3, no. 2, 1 February 2009 (2009-02-01), pages 148-154, XP055799170, DE ISSN: 1862-8346, DOI: 10.1002/prca.200800153

## Description

### FIELD

The present invention relates to a method for determining coronaviruses, in particular to methods wherein the determining is performed using lectins adapted to bind coronavirus glycoproteins. The invention relates also to kits for the method.

### BACKGROUND

In December 2019, a number of pneumonia cases emerged due to an unknown cause in the city of Wuhan, in People's Republic of China. Investigation into the basis of the outbreak revealed that a novel coronavirus species, initially named the 2019-nCoV, subsequently termed SARS-CoV-2, was the cause of the outbreak. The infection rapidly spread throughout the world, reaching multiple countries and continents, including Asia, Europe, and North America. Confirmed SARS-CoV-2 cases reported symptoms of respiratory illness with fever, cough, shortness of breath and myalgia. While many patients present with mild symptoms, increased risk of adverse outcomes have been reported for people with advanced age and co-morbidities such as diabetes and heart disease.

Laboratory assays for coronaviruses are mainly based on viral nucleic acid detection. In the first step, viral genome must be extracted and purified from the sample, the single-stranded RNA genome is reverse-transcribed into complementary cDNA, which is then amplified into a detectable level using polymerase chain reaction (PCR) or an applicable isothermal nucleic acid amplification technique. Nucleic acid amplification techniques are highly sensitive since, in theory, a single copy of a given nucleic acid sequence can accumulate into a detectable level after an adequate number of amplification cycles. In addition, they are typically very specific because the amplification primers and detection probes can be designed for sequences which are specific for the unique sequences of the detection target. On the other side, their recognized deficiencies include the requirement for a relatively pure starting material (purification of RNA or DNA from the sample required), as well as a certain level of complexity and the analysis time. Such factors impede the capacity of the laboratory, limiting the throughput and the number of samples a laboratory can analyze, which is harshly encountered in outbreaks such as the COVID-19. In addition, PCR-based methods are also sensitive to contaminations, even a single copy of the virus in a clean sample can cause a false positive result. Currently, clinical laboratories working with SARS-CoV-2 detection with PCR are prone to contaminations, due to the high volume of samples that are positive for SARS-CoV-2. Thus, some samples have to be re-run to verify the results, which further limits the throughput.

There are also serological tests for SARS-CoV-2 detection, i.e., tests measuring titer of the blood IgM / IgG raised against the virus. However, there is typically a delay in antibody generation after infection (3-5 days), thus a new infection cannot be detected with these tests.

A further challenge is that the COVID-19 coronavirus can survive from hour to several days on frequently touched surfaces. It has also been found that this virus can hang out as droplets in the air for up to three hours before they fall.

Document WO9106311 discloses lectin-based detection method for RNA-viruses. It does not relate however to COVID-19 coronavirus.

Accordingly, there is a need for further methods for determining coronaviruses.

### SUMMARY

The present invention is based on the observation that coronaviruses can be determined from surfaces in the aid of lectins. It was observed that the lectins are also suitable for determining coronaviruses from body fluid samples.

Accordingly, it is an object of the present invention to provide a method for determining SARS-CoV-2 coronavirus from a sample, the method comprising
a) providing
   i. a solution comprising
      ∘ one or more components adapted to neutralize coronavirus,
      ∘ one or more components adapted to expose glycoproteins of the coronavirus,
   ii. a conjugate comprising
      ∘ a first lectin adapted to bind the glycoproteins, wherein the binding is selective,
      ∘ a signal generation means,
   iii. a probe comprising a detection zone comprising an immobilized second lectin adapted to bind the glycoproteins, wherein the binding is selective,
b) immersing the sample to the solution,
c) exposing the conjugate to the solution comprising the sample,
d) exposing the detection zone to the solution comprising the sample and the conjugate,
e) preferably removing unbound material from the detection zone,
f) detecting signal derived from the signal generation means on the detection zone, and
g) determining the coronavirus in the sample based on the detecting,
wherein the first lectin and the second lectin are selected from O-glycan binding lectins.

It is also an object of the present invention to provide a method for determining SARS-CoV-2 coronavirus from a sample, the method comprising the steps of:
a) providing a solution comprising
   ∘ one or more components adapted to neutralize coronavirus,
   ∘ one of more components adapted to expose glycoproteins of the coronavirus,
b) immersing the sample to the solution,
c) determining level of sialic acids and/or O-glycans in the solution by using a lectin array comprising one or more lectins adapted to bind selectively to O-glycans of glycoproteins wherein the binding is indicative to presence of coronavirus in the sample.

It is also an object of the present invention to provide use of kit for determining SARS-CoV-2 coronavirus from a sample according to any one of claims 1-8, the kit comprising
i. a solution comprising
   ο alcohol preferably selected from methanol, ethanol, and isopropanol, more preferably ethanol and isopropanol and mixture thereof, most preferably ethanol,
   ∘ one of more components selected from sodium dichloroisocyanurate, peroxyacetic acid, sodium dodecyl sulfate, and benzalkonium chloride
ii. a conjugate comprising
   ∘ a first lectin, and
   ∘ a detectable label such as colloidal gold,
iii. a probe comprising a detection zone comprising an immobilized second lectin, and optionally
iv. a solution comprising N-acetyl-L-cysteine,
wherein the first lectin and the second lectin is a O-glycan binding lectin selected from a group consisting of HMA, SAMB SNA-I, Agaricus bisphorus lectin (ABL), Amaranthus caudatus (ACL), Bauhinia forficate (BfL), Bouhinia purpurea (BPA), Grriffonia (Bandeiraea) simplicifolia (GS-IV), soybean agglutinin lectin (SBA)and Glycine max (soybean) lectin (SAB).

It is also an object of the present invention to provide use of a kit for determining SARS-CoV-2 coronavirus from a sample in a method according to any one of claims 9-13, the kit comprising
i. a solution comprising
   ο alcohol preferably selected from methanol, ethanol, and isopropanol, more preferably ethanol and isopropanol and mixture thereof, most preferably ethanol,
   ∘ one of more components selected from sodium dichloroisocyanurate, peroxyacetic acid, sodium dodecyl sulfate, and benzalkonium chloride,
ii. a lectin array comprising one or more lectins selected from a group consisting of HMA, SAMB SNA-I, Agaricus bisphorus lectin (ABL), Amaranthus caudatus (ACL), Bauhinia forficate (BfL), Bouhinia purpurea (BPA), Grriffonia (Bandeiraea) simplicifolia (GS-IV), soybean agglutinin lectin (SBA), and Glycine max (soybean) lectin (SAB), and optionally
iii. a solution comprising N-acetyl-L-cysteine.

Further objects of the present invention are described in the accompanying dependent claims.

Exemplifying and non-limiting embodiments of the invention, both as to constructions and to methods of operation, together with additional objects and advantages thereof, are best understood from the following description of specific exemplifying embodiments when read in connection with the accompanying drawings.

The verbs "to comprise" and "to include" are used in this document as open limitations that neither exclude nor require the existence of un-recited features. The features recited in the accompanied depending claims are mutually freely combinable unless otherwise explicitly stated. Furthermore, it is to be understood that the use of "a" or "an", i.e., a singular form, throughout this document does not exclude a plurality.

### BRIEF DESRCIPTION OF THE DRAWINGS

Figure 1 illustrates a schematic view of a lipid/protein layer of a coronavirus **(A** = nucleocapsid protein and RNA; **B** = phospholipid bilayer and membrane proteins; **C** = envelope protein; **D** = spike protein; **E** = hemagglutinin esterase dimer, **F** = glycan linked to the spike protein),
figures 2-5 illustrate exemplary non-limiting steps of the method of the present invention,
figure 6 shows mass spectrum of N-glycans for SARS-CoV-2 S-protein,
figure 7 shows mass spectrum of O-glycans for SARS-CoV-2 S-protein,
figure 8 shows detection of coronavirus spike protein on lateral flow strip spotted with SARS-CoV-2 spike protein and gold conjugated specific lectin (A); no lectin (B); non-relevant glycoprotein (C), and
figure 9 shows nitrocellulose test strips wherein SARS-CoV-2 Spike protein has reacted with different lectins having specificities relevant to glycosylation pattern on the protein.

### DESCRIPTION

Coronaviruses (CoVs) contain a relatively large genome of approximately 30kb, which encodes four structural proteins: spike (S), nucleocapsid (N) envelope (E), and membrane (M). The S protein mediates virus attachment to the host cell receptor and fusion with the cell membrane. The **N** protein interacts with the viral RNA to form the ribonucleoprotein. The E protein is involved in virion assembly and envelope formation, and the M protein drives the virus budding process.

The CoV S protein is a glycoprotein composed of two functional subunits: S1 subunit mediates binding to the host cell receptor and the S2 subunit mediates the fusion of the viral and cellular membranes. Between the SARS-CoV-2 and SARS-CoV Urbani strain S proteins, there is a 76% amino acid sequence identity. The S subunits are extensively glycosylated with N-linked glycans which are important for proper folding and for accessibility to host proteases and neutralizing antibodies.

The CoV spike (S) protein is highly glycosylated with N-linked glycans and this glycosylation is critical for infectivity, viral particle formation and immune evasion. For example, SARS-CoV-2 S protein shows 76% sequence similarity to SARS-CoV S protein, both of which contain dozens of N-linked glycosylation sites in their sequence. SARS-CoV-2 may contain unique O-liked glycans that will make it simpler to detect and specific lectins that will support the test selectivity. The dense O-liked glycans could create a mucin that protects epitopes or key residues on the SARS-CoV-2 spike protein. Several viruses utilize mucin-like domains as glycan shields involved immune-evasion.

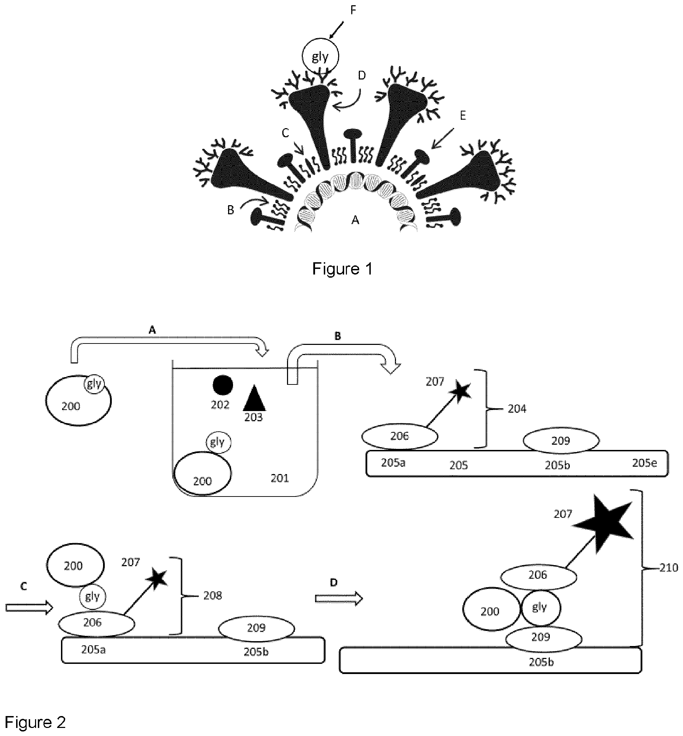

Figure 1 illustrates a schematic view of a lipid/protein layer of a coronavirus to be hydrolyzed and at least partially broken by the solution used in the method of the present invention. The protruding glycans F of the spike protein **D** are the target glycoproteins determined by the method.

According to one embodiment the present invention concerns a method for determining coronavirus from a sample, the method comprising
a) providing
   i. a solution comprising
      ∘ one or more components adapted to neutralize coronavirus,
      ∘ one of more components adapted to expose glycoproteins of the coronavirus,
   ii. a conjugate comprising
      ∘ a first lectin adapted to bind the glycoproteins, wherein the binding is selective
      ∘ a signal generation means,
   iii. a probe comprising a detection zone comprising an immobilized second lectin adapted to bind the glycoproteins, wherein the binding is selective,
b) immersing the sample to the solution,
c) exposing the conjugate to the solution comprising the sample,
d) exposing the detection zone to the solution comprising the sample and the conjugate,
e) preferably removing unbound material from the detection zone,
f) detecting signal derived from the signal generation means on the detection zone, and
g) determining the coronavirus in the sample based on the detecting,
wherein the first lectin and the second lectin is

*O*-glycan binding lectins. The detected signal is an indication of presence of coronavirus in the sample.

As defined herein a probe is a device to obtain information for diagnostic purposes. Exemplary probes are a test strip and a glass slide.

According to one embodiment the first lectin and the second lectin are selected from sialic acid binding lectins. According to another embodiment the first lectin and the second lectin are selected from O-glycan binding lectins. According to still another embodiment the first lectin and the second lectin are selected from lectins which bind sialic acids and O-glycans. The sialic acid binding lectins are preferably selected from Sambucus nigra I lectin (SNA-I), Polyporus squamosus lectin (PSL1A) Agrocybe cylindracea lectin (ACG), Homarus americanus lectin (HMA), Sambucus sieboldiana lectin (SAMB), Lotus tetragonolobus lectin (Lotus), Sambucus nigra agglutinin II lectin (SNA-II), and Limulus Polyphemus lectin (LPA) more preferably ACG and HMA, most preferably ACG. In the literature, an alternative name for SNA is Elderberry bark lectin (EBL).

The O-glycan binding lectins are preferably selected from a group consisting of HMA, SAMB SNA-I, Agaricus bisphorus lectin (ABL), Amaranthus caudatus lectin (ACL), Bauhinia forficate lectin (BfL), Bouhinia purpurea lectin (BPA), Grriffonia (Bandeiraea) simplicifoia lectin (GS-IV), Artocarpus integrifolia lectin (Jacalin), soybean agglutinin lectin (SBA), and Glycine max (soybean) lectin (SAB) more preferably from ABL and ACL, most preferably ABL.

Lectins which bind both sialic acid and O-glycans are preferably selected from HMA, SAMB, and SNA-I.

Figure 2 shows an exemplary non-limiting method for determining coronavirus from a sample. In Step A, a sample comprising coronavirus 200 is immersed to a solution 201 comprising a component 202 adapted to neutralize the coronavirus and a component 203 adapted expose glycoproteins (gly) of the coronavirus.

In step B a conjugate 204 embedded on a first part 205a of a probe such as a test strip 205 is exposed to the solution comprising the exposed glycoprotein. The conjugate comprises a first lectin 206 adapted to bind selectively the glycoprotein, and a signal generation means 207.

Step C shows a situation where the glycoprotein has bound to the conjugate and a complex 208 has formed. The solution comprising the complex migrates towards the second part 205e of the probe through the detection zone 205b. The detection zone comprises a second lectin 209 which is also adapted to bind selectively the glycoprotein and thus also the complex 208. The second lectin is immobilized on the detection zone.

In step D a new complex 210 bound to the probe via the second lectin has formed. Signal derived from the signal generation means on the detection zone is an indication of presence of coronavirus in the sample. According to a preferable embodiment, unbound material is removed from the detection zone before detecting the signal. The removing can be done e.g., by washing the probe with a wash solution. An exemplary wash solution is saline such as PBS.

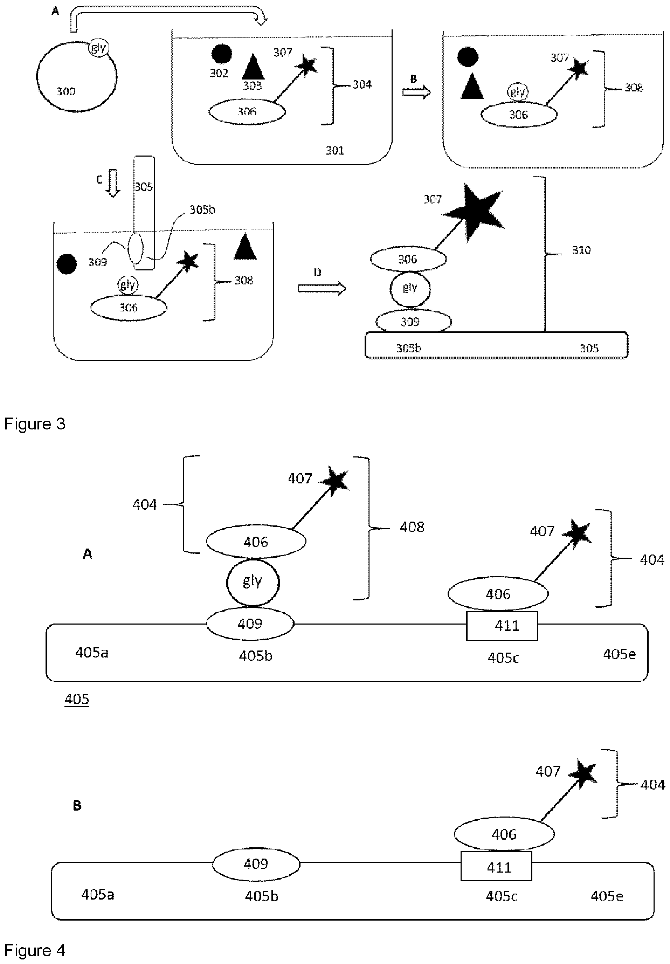

Figure 3 shows another exemplary non-limiting method for determining coronavirus from a sample. The coronavirus 300 has been omitted from steps B-D of the figure for clarity. In Step A, the sample comprising coronavirus 300 is immersed to a solution 301 comprising a component 302 adapted to neutralize the coronavirus, a component 303 adapted expose glycoproteins (gly) of the coronavirus, and a conjugate 304 comprising a first lectin 306 and a signal generation means 307. The first lectin is adapted to bind the exposed glycoprotein.

Step B shows a situation where the glycoprotein has bound to the first lectin of the conjugate and in the solution and a complex 308 has formed.

In step C, a probe 305 comprising an immobilized second lectin 309 on a detection zone 305b has been immersed to the solution. The second lectin is able to bind the glycoprotein of the complex 308. The binding is selective.

In step D the probe has been removed from the solution and the new complex 310 bound to the probe via the second lectin has formed. Signal derived from the signal generation means on the detection zone is an indication of presence of coronavirus in the sample. According to a preferable embodiment, unbound material is removed from the detection zone before detecting the signal. The removing can be done e.g., by washing the probe with a wash solution. An exemplary wash solution is saline such as PBS.

According to a preferable embodiment the method comprises also a quality control to verify the validity of the determining. The quality control can be done using a probe such as a test strip comprising a detection zone comprising the immobilized second lectin adapted to bind selectively a glycoprotein of a coronavirus, and a control zone comprising an immobilized molecule adapted to bind any lectins, i.e., the binding is unselective.

An exemplary probe 405 comprising a first part 405a, a detection zone 405b a control zone 405c and a second part 405e suitable for the method is shown in figure 4. A solution comprising the sample and a conjugate 404 is typically applied on the first part of the probe and allowed to migrate towards the second part 405e of the probe through the detection zone and the control zone. The detection zone comprises an immobilized second lectin 409 adapted to bind selectively the complex 408, while the control zone comprises an immobilized molecule 411 adapted to bind unselectively the conjugate 404, i.e., independently on the structure of the first lectin 406. Thus, the immobilized molecule 411 binds the conjugate 404 irrespectively of the presence of the coronavirus in the sample.

Exemplary suitable molecule is an antibody adapted to bind the first lectin to its "common parts" i.e., not the glycan-recognizing sites that are shielded by the bound virus. Further suitable molecules are polymers and macromolecules containing carbohydrates. Since the control zone captures the conjugate through such parts that do not participate in the virus recognition, both positive and negative samples react identically on the control line.

Figure 4 (top) shows a situation where signal of the signal generation means 407 is detected from the detection zone and the control zone. According to a preferable embodiment the method is typically configured so that there is an excess of conjugate. Thus, some of the conjugate will pass through the detection zone and will be captured on the control. Also, if the detection zone did not have capacity to bind all the complex 408, some of them migrate through and are captured on the control zone. The presence of signal in both zones is an indication of presence of coronavirus in the sample.

Figure 4 (bottom) shows a situation where signal of the signal generation means 407 can be detected from the control zone only. This is an indication that the sample does not include coronavirus.

When the signal of the signal generation means 407 is not detected on the control zone the determination has not been successful and should be repeated. However, in some instances signal derived from the detection zone alone appears, and the test is regarded as positive. This may happen if the virus is very abundant in the sample, it depletes all the conjugate, and the immobilized lectin in the detection zone has the capacity to bind the formed complex 408 completely so that nothing passes through to the control line.

The one or more components adapted to neutralize the coronavirus are preferably selected from alcohols although also other components, such as hydrogen peroxide, various solvents, detergents, and cross-linking agents could be used. Exemplary alcohols are methanol, ethanol, and isopropanol. Preferable alcohols are ethanol and isopropanol. A particular alcohol is ethanol. The amount of alcohol of the solution should be high enough to neutralize the coronavirus. An exemplary amount of ethanol is 70% by volume.

The one or more components adapted to expose glycoproteins of the coronavirus are preferably selected from sodium dichloroisocyanurate, peroxyacetic acid, sodium dodecyl sulfate, and benzalkonium chloride. An exemplary concentration of sodium dichloroisocyanurate is 50 mg/L. An exemplary concentration of peroxyacetic acid is 3000 ppm or more. An exemplary concentration of sodium dodecyl sulfate is 0.23 %, w/v. An exemplary concentration of benzalkonium chloride is 0.13 % v/v.

The first lectin is conjugated to a signal generation means. The signal generation means is typically a label, preferably a visually detectable label. In some embodiments, the lectin is conjugated to the visually detectable label covalently. In some other embodiments, the lectin is conjugated to the visually detectable label non-covalently. The covalent and non-covalent binding should be high enough to warrant the stability of the conjugate in the solution and during applying the solution on the probe.

The most commonly used signal generation means in lateral flow immunoassays are gold nanoparticles or latex beads. These particles produce a colored readout which requires no development process for visualization. Fluorescent labels, enzymes, other colloidal metals, and magnetic particles can also be employed.

A particular detectable label is colloidal gold. Further labels suitable for lateral flow assays have been disclosed by Badir et al. (Trends. Anal. Chem., 82, 2016, pp. 286-306).

In the context of embodiments of the present invention, the term "visible" refer to a visual signal that can be detected by the naked eye (visible light which a human eye can perceive), without the use of additional machinery or processes. In the context of embodiments of the present invention, a visible signal is a change in a color intensity of a certain object or an area thereon, relative to the color that has been characteristic to the object or area prior to the change. A change can also be assessed in comparison to the background of the object or area, and in comparison, to the surrounding of the object or area.

There exists plurality of labels suitable for the present technology which have better detection sensitivity than labels which can be detected with naked eye. Exemplary labels are luminescent lanthanide(III) chelates which generate time resolved luminescence. However, their detection requires the use of dedicated instruments.

The first lectin is preferable selected from a lectin capable of binding sialic acid residues of glycoproteins. This is because the CoV spike typically includes these residues. The first lectin is preferable selected from SNA-I, PSL1A, ACG, HMA, SAMB, Lotus, SNA-II, and LPA, more preferably ACG and HMA, most preferably ACG.

The second lectin is also preferable selected from a lectin capable of binding sialic acid residues of glycoproteins. The second lectin is preferable selected from a group consisting of SNA-I, PSL1A, ACG, HMA, SAMB, Lotus, SNA-II, and LPA, more preferably ACG and HMA, most preferably ACG.

According to another embodiment the first lectin and the second lectin is adapted to target O-linked glycans of the coronavirus. According to this embodiment the first lectin and the second lectin is preferably selected from a group consisting of HMA, SAMB SNA-I, ABL, ACL, BfL, BPA, GS-IV, Jacalin, SBA, and SAB, more preferably ABL and ACL, most preferably ABL. Lectins which bind both sialic acid and O-glycans are preferably selected from HMA, SAMB, and SNA-I.

The second lectin is immobilized on the probe. The probe comprises preferably a porous matrix. Various "printing" techniques have been proposed for application of liquid reagents to probes comprising porous matrices, e.g., micro-syringes, pens using metered pumps, direct printing, and inkjet printing, and any of these techniques can be used in the present context. To facilitate manufacture, the matrix can be treated with the reagents and then subdivided into smaller portions, e.g., small narrow strips each embodying the required reagent-containing zones, to provide a plurality of identical carrier units. The lectin is preferably permanently immobilized on the probe and is therefore not mobile in the moist state e.g., when the solution is applied on the probe.

According to a particular embodiment the first lectin and the second lectin are same lectins.

The conjugate comprising the first lectin may be part of the solution or it may be embedded to the probe as shown in figures 2 and 3, respectively.

According to one embodiment the sample comprises material obtained from a surface suspected to be contaminated by coronavirus.

There are different ways to obtain the sample from the surface. According to a particular embodiment the sample is adsorbed to a wiping means, and the wiping means comprising the sample is immersed to the solution. Exemplary wiping means comprise swab, sponge, cloth, and paper towel. A particular wiping means is nasopharyngeal swab.

According to another embodiment the sample comprises saliva or sputum obtainable from a subject suspected to be infected with coronavirus. The sample is immersed to the solution and the solution comprising the sample is processed as disclosed above.

When the sample comprises saliva or sputum, its viscosity may be high. The high viscosity may prevent or at least disturb elution of the sample on the probe. Accordingly, it is preferable to expose the sample comprising saliva or sputum to N-acetyl-L-cysteine, since this compound was found to decrease sample viscosity. It is suspected that the compound is able to disintegrate mucous proteins.

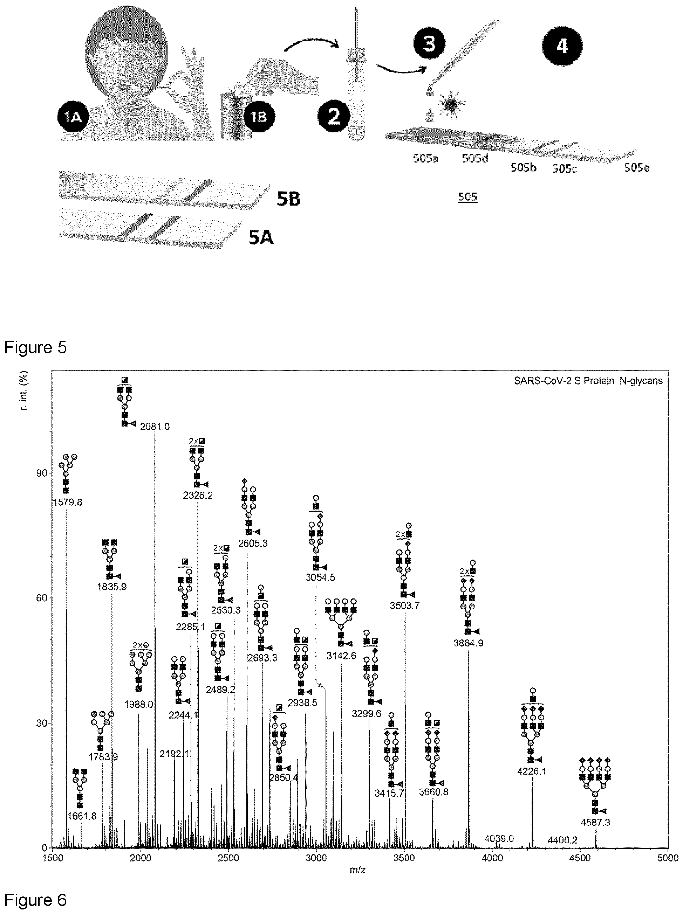

Figure 5 shows an exemplary workflow of the method of the present invention utilizing a lateral flow assay using a probe 505 in a form of a lateral flow test strip. The probe comprises a first part 505a where the sample is applied, a detection zone 505b comprising an immobilized first lectin adapted to bind selectively coronavirus glycoprotein, a control zone 505c comprising an immobilized molecule adapted to bind unselectively lectins independently on their structures. The probe comprises also a zone 505d comprising an embedded conjugate of the first lectin and a signal generation means. The method comprises the following steps:
Step 1. The sample is collected with a cotton swab from the back of subject's throat or nose (1A) or from surfaces (1B) of equipment, devices, skin, or the like.
Step 2. The sample adsorbed on the cotton swab is immersed to a solution adapted to inactivate the coronavirus and to expose the glycoproteins of the coronavirus for detection.
Step 3. The solution comprising the exposed glycoproteins is dispensed on the first part 505a of the probe 505 by a pipette. The solution comprising the sample absorbs to the probe.
Step 4. The solution comprising the sample migrates from the first part 505a towards the second part 505d though a zone 505d comprising an embedded conjugate comprising a visually detectable label. The glycoprotein of the sample, if present, binds to the conjugate and a complex is formed. The complex migrates through the detection zone 505b and the control zone 505c towards the second part 505e. When the coronavirus is present, the second lectin, which is immobilized on the detection zone, captures the complex and a color appears on the detection zone.

When the coronavirus is not present, the detection zone remains invisible, but the color development on the control zone ensures that the test is performed properly.

Exemplary test strips comprising a negative and positive result are shown as 5A - coronavirus is not present (negative) color appears in control zone.

5B - coronavirus present in the sample (positive) color appears in detection zone and control zone.

According to another embodiment the present invention concerns a method for steps of:
a) providing a solution comprising
   ∘ one or more components adapted to neutralize coronavirus,
   ∘ one of more components adapted to expose glycoproteins of the coronavirus,
b) immersing the sample to the solution,
c) determining level of O-glycans in the solution by using a lectin array comprising one or more lectins adapted to bind selectively O-glycans of glycoproteins wherein the is indicative to presence of coronavirus in the sample.

Standard techniques of protein microarray technology can be applied to analyze the sialic acids and/or O-glycans of the coronavirus. In such microarrays, lectins are immobilized on a solid support, such as a slide, in a high spatial density. Each lectin may be arrayed at several concentrations and in replicates on each slide. The concentration ranges may be tailored for each of the lectins and calibrated to provide a linear response within the same range, regardless of the affinity of the lectin. A sample is applied to the array, and its binding pattern is detected by a label, such as a fluorescent label, a radioactive label, or a chemiluminescent label, which is placed either on the coronavirus itself or on the lectin directed toward the sialic acid residues and/or O-glycans of the coronavirus. Streptavidin may be used for detecting biotinylated samples.

Suitable microarray substrates include, but are not limited to, glass, silica, aluminosilicates, borosilicates, metal oxides such as alumina and nickel oxide, gold, various clays, nitrocellulose, or nylon. In some embodiments a glass substrate is preferred. In other embodiments, the substrate may be coated with a compound to enhance binding of the lectin to the substrate. In some further embodiments, lectins have been arrayed on a nitrocellulose membrane-coated glass slide. In some still further embodiments, one or more control lectins are also attached to the substrate.

In some embodiments, a commercially available lectin array, which encompasses one standard glass slide, which is spotted with 8 wells of identical lectin arrays, may be employed. Each lectin, together with the positive controls is arrayed in duplicate. The slide comes with an 8-well removable gasket which allows for the process of 8 samples using one slide. Four-slide slides can be nested into a tray, which matches a standard microplate and allows for automated robotic high throughput process of 64 arrays simultaneously.

The sialic acid binding lectins of the array comprise preferably one or more from the group consisting of Sambucus nigra I (SNA-I), Polyporus squamosus lectin (PSL1A), Agrocybe cylindracea lectin (ACG), Homarus americanus lectin (HMA), Sambucus sieboldiana lectin (SAMB), Lotus tetragonolobus lectin (Lotus), Sambucus nigra agglutinin II lectin (SNA-II), and Limulus Polyphemus lectin (LPA).

The O-glycan binding lectins of the array comprise preferably one or more from the group consisting of HMA, SAMB SNA-I, ABL, ACL, BfL, BPA, GS-IV, Jacalin, and SAB.

According to one embodiment the sample comprises saliva or sputum obtainable from a subject suspected to be infected by the coronavirus. According to another embodiment the sample comprises material obtained from a surface suspected to be contaminated by the coronavirus.

According to one embodiment the sample is adsorbed on a wiping means, and wherein the step b) comprises immersing the wiping means comprising the adsorbed sample to the solution.

The one or more components adapted to neutralize the coronavirus is preferably alcohol preferably selected from methanol, ethanol, and isopropanol, preferably ethanol and isopropanol and mixture thereof, most preferably ethanol. The one of more components adapted to expose glycoproteins of the coronavirus are typically selected from sodium dichloroisocyanurate, peroxyacetic acid, sodium dodecyl sulfate, and benzalkonium chloride.

The coronavirus is selected from group consisting of SARS-CoV, MERS-CoV and SARS-CoV-2, preferably SARS-CoV-2.

It is known that O-linked glycosylation takes place to the hydroxy group of amino acids serine or threonine in the protein. The first core monosaccharide binding covalently to the hydroxy is N- Acetyl-galactosamine. A mutation in the virus genome (RNA in coronavirus) generates a new variant of virus. However, as long as the mutation does not affect the codons for serine (AGT or AGC) or threonine (ACC or ACA or ACG), those amino acids stay in the protein structure and get glycosylated as well. Accordingly, as long as this is the case the method of the present invention is suitable also for detection variants or mutated strains of the virus.

When the sample comprises saliva or sputum, the method comprises preferably exposing the sample to N-acetyl-L-cysteine.

According to another embodiment the present invention concerns a kit for determining coronavirus from a sample, the kit comprising
i. a solution comprising
   ∘ one or more components adapted to neutralize the coronavirus
   ∘ one of more components adapted to expose glycoproteins of the coronavirus
ii. a conjugate comprising
   - a first lectin adapted to bind the glycoproteins, wherein the binding is selective and
   - a signal generation means, and
iii. a probe comprising a detection zone comprising an immobilized second lectin adapted to bind the glycoproteins, wherein the binding is selective wherein the first lectin and the second lectin is *O*-glycan binding lectins.

According to one embodiment the first lectin and the second lectin is selected from sialic acid binding lectins. According to another embodiment the first lectin and the second lectin is selected from *O*-glycan binding lectins. According to still another embodiment the first lectin and the second lectin is selected from lectins which bind sialic acid s and O-glycans. The sialic acid binding lectins of the kit are preferably selected from SNA-I, SNA-II, PSL1A ACG, HMA, SAMB, Lotus, and LPA, more preferably ACG and HMA, most preferably ACG. The *O*-glycan binding lectins of the kit are preferably selected from a group consisting of HMA, SAMB SNA-I, ABL, ACL, BfL, BPA, GS-IV, Jacalin, and SAB. Lectins which bind both sialic acid and O-glycans are preferably selected from HMA, SAMB, and SNA-I, more preferably from ABL and ACL, most preferably ABL.

The one or more components of the kit adapted to neutralize the coronavirus are preferably selected from alcohols. Exemplary alcohols are methanol, ethanol, and isopropanol. Preferable alcohols are ethanol an isopropanol. A particular alcohol is ethanol. The amount of alcohol of the solution should be high enough to neutralize the coronavirus. An exemplary amount of alcohol is 70% by volume.

The one or more components of the kit adapted to expose glycoproteins of the coronavirus are preferably selected from sodium dichloroisocyanurate, peroxyacetic acid, sodium dodecyl sulfate, and benzalkonium chloride. An exemplary concentration of sodium dichloroisocyanurate is 50 mg/L. An exemplary concentration of peroxyacetic acid is 3000 ppm or more. An exemplary concentration of sodium dodecyl sulfate is 0.23 %, w/v. An exemplary concentration of benzalkonium chloride is 0.13 % v/v.

The first lectin of the kit is preferable selected from a lectin capable of binding sialic acid residues of glycoproteins. This is because the CoV spike typically includes these residues. The first lectin is preferable selected from SNA-I, SNA-II, PSL1A ACG, HMA, SAMB, Lotus, and LPA.

The second lectin of the kit is also preferable selected from a lectin capable of binding sialic acid residues of glycoproteins. The second lectin is preferable selected from SNA-I, SNA-II, PSL1A ACG, HMA, SAMB, Lotus, and LPA. preferably ACG and HMA, most preferably ACG.

According to another embodiment the first lectin and the second lectin of the kit is adapted to target O-linked glycans of the coronavirus. According to this embodiment the firs lectin and the second lectin is preferably selected HMA, SAMB SNA-I, ABL, ACL, BfL, BPA, GS-IV, Jacalin, and SAB, preferably ABL and ACL, most preferably ABL. Lectins which bind both sialic acid and O-glycans are preferably selected from HMA, SAMB, and SNA-I.

According to a particular embodiment the first lectin and the second lectin are same lectins.

According to a preferable embodiment the probe of the kit comprises also a control zone comprising an immobilized molecule adapted to bind the conjugate, wherein the binding is unselective.

The second lectin and the molecule adapted to bind the first lectin of the conjugate unselectively are immobilized on the probe. The probe comprises preferably a porous matrix. Various "printing" techniques have been proposed for application of liquid reagents to probes comprising porous matrices, e.g., micro-syringes, pens using metered pumps, direct printing, and inkjet printing, and any of these techniques can be used in the present context.

According to a preferable embodiment the kit comprises a wiping means and/or a wash solution adapted to remove unbound material from the probe. Exemplary wiping means are swab and a sponge. Exemplary wash solution is saline such as phosphate buffered saline.

According to another embodiment the present invention concerns a kit for determining coronavirus from a sample, the kit comprising
i. a solution comprising
   ∘ one or more components adapted to neutralize the coronavirus,
   ∘ one of more components adapted to expose glycoproteins of the coronavirus, and
ii. a lectin array comprising one or more lectins adapted to bind selectively to *O*-glycans of glycoproteins.

The sialic acid binding lectins of the array of the kit comprise preferably one or more of Sambucus nigra I (SNA-I), Polyporus squamosus lectin (PSL1A) Agrocybe cylindracea lectin (ACG), Homarus americanus lectin (HMA), Sambucus sieboldiana lectin (SAMB), Lotus tetragonolobus lectin (Lotus), Sambucus nigra agglutinin II (SNA-II), and Limulus Polyphemus.

The O-glycan binding lectins of the array of the kit comprise preferably one or more of HMA, SAMB SNA-I, Agaricus bisphorus lectin (ABL), Amaranthus caudatus (ACL), Bauhinia forficate (BfL), Bouhinia purpurea (BPA), Grriffonia (Bandeiraea) simplicifolia (GS-IV), Artocarpus integrifolia lectin (Jacalin), and Glycine max (soybean) (SAB),

The kit comprises also one or more components adapted to neutralize the coronavirus such as alcohol which is preferably selected from methanol, ethanol, and isopropanol, more preferably ethanol and isopropanol and mixture thereof, most preferably ethanol.

The kit comprises also more components adapted to expose glycoproteins of the coronavirus which are preferably selected from sodium dichloroisocyanurate, peroxyacetic acid, sodium dodecyl sulfate, and benzalkonium chloride.

The kit comprises preferably also a solution comprising N-acetyl-L-cysteine.

### EXPERIMENTAL

### Ability of lectins to bind SARS-CoV-2 through its spike proteins S1 and S2

Lectin binding assay was performed for spike-proteins (R) S1 and S2 separately. The analyses were performed on lectin-arrays consisting of 95 different lectins immobilized in spots on a glass slide. SARS-CoV-2 spike-proteins were biotinylated and dialyzed against phosphate-buffered saline (PBS). Biotinylated spike proteins (R) S1 and S2 were added on separate lectin array and the detection of bound proteins was enabled by fluorescently labelled streptavidin. The array was scanned to illuminate and quantify the spots with bound S1 and S2. The inter-array variations in fluorescent signals were normalized between the arrays based on the signals of the positive control spots. The background value was subtracted from the lectin signals. Value of >0 indicates binding of the S1 or S2 protein to the given lectin and indicates the presence of lectin-specific glycan residues on S1 and S2. The relative binding by each lectin was determined by dividing the lectin signal by the median of all lectin signals of the same protein. Table 1 shows that several sialic acid-binding lectins and O-glycan-binding lectins are binding S1 and S2.

A value above 1 indicates higher-than-median binding to the given lectin and value below 1 means lower-than-median binding. The relative binding in the table indicates the ratio of one lectin's signal to the median of all lectins' signal.

The sample spike protein was analyzed on a spotted array of 95 different lectins using a fluorescent measurement. The common background signal level was subtracted from all signals. If after the subtraction the signal of a given lectin became zero or less, there was no binding of the spike protein to that lectin. Any signal above zero (net signal in the table) indicates binding of spike protein to that lectin - the higher, the more/stronger binding. A median of net signals was calculated and each lectin's signal was compared to the median → relative binding.

In the table, relative binding of 1.0 means "medium/average" degree of binding. Relative binding less than 1.0 means "lower-than-average" binding, but still there was binding and recognition taking place with lectin as far as the net signal was >0.

**Table 1**

| **Lectin** | **Name / Origin** | **Carbohydrate Specificity** | **Specificity** | | **S1** | | **S2** | |
|---|---|---|---|---|---|---|---|---|
| | | | | | **Net Signal** | **Relative binding*** | **Net Signal** | **Relative binding *** |
| | | | **Sialic acid** | **O-glycan** | | | | |
| **ACG** | Agrocybe cylindracea lectin | α2-3 Sialic Acid | **X** | | 134 803 | 1,6 | 16 816 | 1,4 |
| **LPA** | Limulus polyphemus | Sialic Acid (N-Acetylneuraminic acid) | **X** | | 10 757 | 0,1 | 4 828 | 0,4 |
| **PSL1A** | Polyporus squamosus lectin | α2-6 Sialic Acid | **X** | | 32 489 | 0,4 | 2 625 | 0,2 |
| **HMA** | Homarus americanus lectin | N-Acetylneuraminic acid, N-Acetylgalactosamine | **X** | **X** | 113 165 | 1,3 | 5 293 | 0,4 |
| **SAMB** | Sambucus Sieboldiana Lectin | NeuAcα2-6Gal/GalNAc | **X** | **X** | 26 202 | 0,3 | 8 382 | 0,7 |
| **SNA-I** | Sambucus nigra I | NANAα(2,6)GalNAc > GalNAc = Lac > GalNANAα(2,6)Gal | **X** | **X** | 72 899 | 0,9 | 7 713 | 0,6 |
| **ABL** | Agaricus bisporus lectin | galactose-β-1,3-N-cetylgalactosamine, galactose-β-1,3-N-acetylglucosamine | | **X** | 411 167 | 4,9 | 109 252 | 8,8 |
| **ACL** | Amaranthus caudatus | Galβ3GalNAc | | **X** | 223 440 | 2,7 | 56 533 | 4,5 |
| **BfL** | Bauhinia forficata | GalNAc | | **X** | n.d. | n.d. | n.d. | n.d. |
| **BPA** | Bouhinia purpurea | Galβ3GalNAc | | **X** | 54 667 | 0,7 | 12 450 | 1,0 |
| **GS-IV** | Griffonia (Bandeiraea ) simplicifolia | GalNAc | | **X** | n.d. | n.d. | n.d. | n.d. |
| **Jacalin** | Artocarpus integrifolia (Jackfruit) | Galβ3GalNAc | | **X** | 6046 | 0,1 | 5 029 | 0,4 |
| **SBA** | Glycine max (soybean) | α > βGalNAc | | **X** | 68 516 | 0,8 | 9 846 | 0,8 |

| | | | | | | | | |
|---|---|---|---|---|---|---|---|---|
| *Relative to median of all lectins | | | | | | | | |

### Analysis of glycans on coronavirus by mass spectrometry (MS)

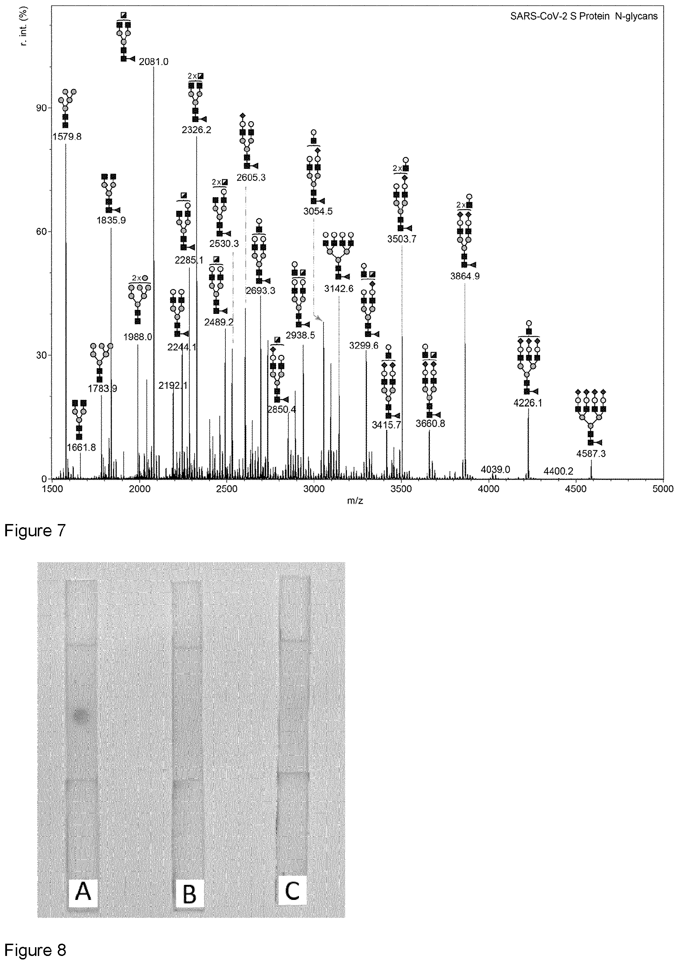

Both N-glycan and O-glycan analyses were performed for 1) S-protein of SARS-CoV-2, 2) S-protein of SARS (Recombinant COVID-19 S protein (R667A) expressed in human cells, and 3) SARS-CoV-2 purified viral lysate (taken from a patient). The proteins were digested with trypsin and the N-glycans were released from the peptides by PNGaseF treatment and purified over C18 columns. The O-glycans were released from the peptides by reduction with sodium borohydride, passed through ion exchange column, and after repeated lyophilization-resuspension cycles purified through C18 column. All purified glycans were permethylated by iodomethane prior to MS analysis. The MS data was acquired with MALDI-TOF method using reflective positive mode. Data was recorded between m/z 500 to 6000 for N-glycans and m/z 500 to 4000 for O-glycans. For each MS N-glycan and O-glycan profiles the aggregation of 20,000 laser shots or more were considered for data extraction. MS spectra are shown in figures 6 and 7

Structures shown in the tables 2 and 3 present the most abundant structures or those compositions that have the highest probability in the tests employed. Structures may vary by additional sialic acid (NANA), phosphate, or fucose. Those additions and subtractions can generate alterations in molecular weight, size, and charge.

Legend for glycan structure symbols used in table 2 and 3 as well as figures 6 and 7 are the following:
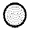 : Galactose (Gal)
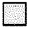 : N-Acetylgalactosamine (GaINAc)
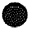 : Glucose (Glc)
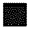 : N-Acetylglucosamine (GIcNAc)
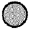 : Mannose (Man)
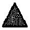 : Fucose (Fuc)
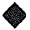 : Sialic Acid (N-acetylneuraminic acid) (Neu5Ac)
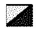 : Hexosamine (GlcNAc or GalNAc)

The arc with adjacent structure = the structure outside the arc belongs to the glycan but its positions is not specified.

**Table 2. Relevant N-glycans detected on S-protein of SARS-CoV-2.**

| | | **N-glycans** | |
|---|---|---|---|
| **m/z** | **Intensity** | **Relative Abundance %** | **Proposed structure** |
| **2081.1** | 320 422 | 9.60% | 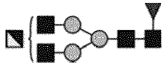 |
| **1579.8** | 272 006 | 8.15% | 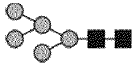 |
| **2326.2** | 230 110 | 6.89% | 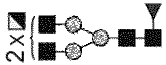 |
| **1835.9** | 204 152 | 6.12% | 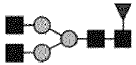 |
| **2285.2** | 147 739 | 4.43 % | 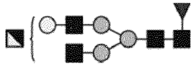 |
| **1988.0** | 109 142 | 3.27% | 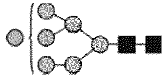 |
| **2693.4** | 105 765 | 3.17% | 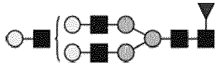 |
| **2244.1** | 100731 | 3.02% | 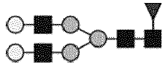 |
| **4226.1** | 11997 | 0.36% | 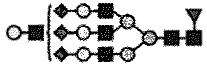 |
| **2396.2** | 4 484 | 0.13% | 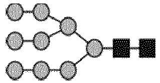 |
| **4587.3** | 2 157 | 0.06% | 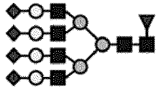 |

**Table 3. Relevant O-glycans detected on S-protein of SARS-CoV-2.**

| | | **O-glycans** | |
|---|---|---|---|
| **m/z** | **Intensity** | **Relative Abundance %** | **Proposed structure** |
| **1852.0** | 83 451 | 42.99 % | 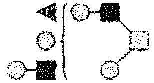 |
| **1256.7** | 24 186 | 12.46 % | 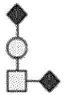 |
| **1606.9** | 18 151 | 9.35 % | 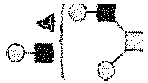 |
| **1705.9** | 16 344 | 8.42 % | 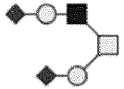 |
| **1432.8** | 15 849 | 8.16 % | 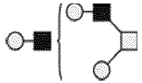 |
| **1647.9** | 6 973 | 3.59 % | 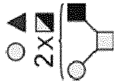 |
| **1576.9** | 5 968 | 3.07 % | 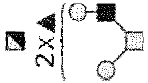 |
| **1344.8** | 4 795 | 2.47 % | 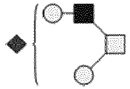 |
| **895.5** | 4 302 | 2.22 % | 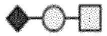 |
| **1361.8** | 3 551 | 1.83 % | 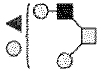 |

### Detection of SARS-CoV-2 Spike protein on a lateral flow strip

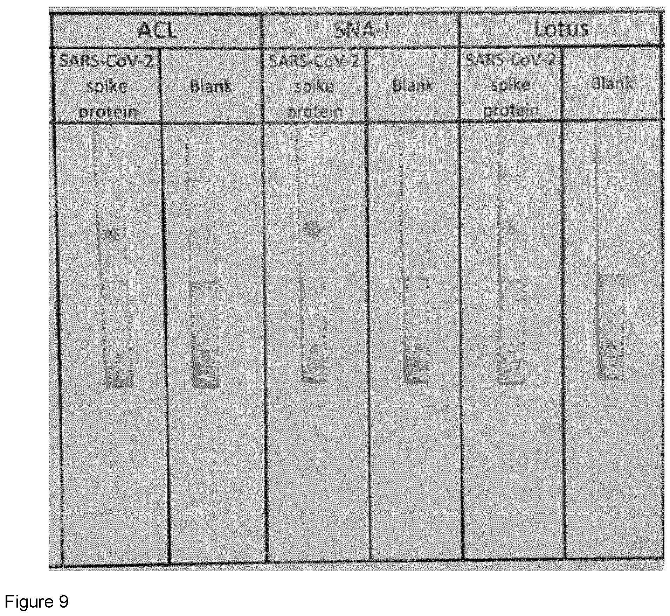

SARS-CoV-2 Spike protein was allowed to react on a nitrocellulose strip with seven different lectins having specificities relevant to glycosylation pattern on the SARS-CoV-2 Spike protein. Results are shown in figures 8 and 9. Visible spot was generated by interaction with gold-conjugated lectins selective to the spike protein. The matching blank strips contain the dilution buffer only (no spike protein present).

The specific examples provided in the description given above should not be construed as limiting the scope and/or the applicability of the appended claims.

## Claims

1. A method for determining SARS-CoV-2 coronavirus from a sample, the method comprising
a) providing
i. a solution (201, 301) comprising
∘ one or more components (202, 302) adapted to neutralize the coronavirus,
∘ one of more components (203, 303) adapted to expose glycoproteins of the coronavirus,
ii. a conjugate (204, 304, 404) comprising
∘ a first lectin (206, 306, 406) adapted to bind the glycoproteins, wherein the binding is selective,
∘ a signal generation means (207, 307, 407), and
iii. a probe (205, 305, 405) comprising a detection zone (205b, 305b, 405b) comprising an immobilized second lectin (209, 309, 409) adapted to bind the glycoproteins, wherein the binding is selective,
b) immersing the sample to the solution,
c) exposing the conjugate to the solution comprising the sample,
d) exposing the detection zone to the solution comprising the sample and the conjugate,
e) preferably removing unbound material from the detection zone,
f) detecting signal derived from the signal generation means on the detection zone, and
g) determining the SARS-CoV-2 coronavirus in the sample based on the detecting, **characterized in that** the first lectin and the second lectin is selected from O-glycan binding lectins.

2. The method according to claim 1 wherein O-glycan binding lectin is selected from a group consisting of Homarus americanus lectin (HMA), Sambucus sieboldiana lectin (SAMB), Sambucus nigra I lectin (SNA-I), Agaricus bisphorus lectin (ABL), Amaranthus caudatus lectin (ACL), Bauhinia forficate lectin (BfL), Bouhinia purpurea lectin (BPA), Griffonia (Bandeiraea) simplicifoia lectin (GS-IV), Artocarpus integrifolia lectin (Jacalin), soybean agglutinin lectin(SBA),and Glycine max (soybean) lectin (SAB), preferably ABL and ACL, most preferably ABL.

3. The method according to claim 1 or 2 wherein the sample comprises saliva or sputum obtainable from a subject suspected to be infected by the coronavirus.

4. The method according to claim 1 or 2 wherein the sample comprises material obtained from a surface suspected to be contaminated by the coronavirus.

5. The method according to any one of claims 1-4 wherein the sample is adsorbed on a wiping means, and wherein the step b) comprises immersing the wiping means comprising the adsorbed sample to the solution.

6. The method according to any one of claims 1-5 wherein the first lectin and the second lectin are similar lectins.

7. The method according to any one of claims 1-6 wherein the sample comprises saliva or sputum, and wherein the method comprises exposing the sample to N-acetyl-L-cysteine.

8. The method according to any one of claims 1-7, wherein the probe (405) comprises a control zone (405c) comprising an immobilized molecule (411) adapted to bind the first lectin wherein the binding is unselective, and wherein the method comprises
h) exposing the control zone to the solution comprising the sample and the conjugate (404),
i) preferably removing unbound material from the control zone,
j) detecting signal derived from the signal generation means on the control zone, and
k) determining the presence or absence of coronavirus in the sample wherein
• detecting signal on the detection zone and on the control zone is an indication of presence of the coronavirus in the sample, and
• absence of signal on the detection zone and detecting signal on the control zone is an indication of the absence of coronavirus in the sample.

9. A method for determining SARS-CoV-2 coronavirus from a sample, the method comprising the steps of:
a) providing a solution comprising
∘ one or more components adapted to neutralize the coronavirus,
∘ one of more components adapted to expose glycoproteins of the coronavirus,
b) immersing the sample to the solution,
c) determining O-glycans in the solution by using a lectin array comprising one or more lectins adapted to bind selectively to O-glycans of glycoproteins wherein the binding is indicative of SARS-CoV-2 coronavirus in the sample.

10. The method according to claim 9 wherein the O-glycan binding lectins of the array comprise one or more of a group consisting of HMA, SAMB SNA-I, Agaricus bisphorus lectin (ABL), Amaranthus caudatus lectin (ACL), Bauhinia forficate lectin (BfL), Bouhinia purpurea lectin (BPA), Grriffonia (Bandeiraea) simplicifoia lectin (GS-IV), Artocarpus integrifolia lectin (Jacalin), soybean agglutinin lectin(SBA), and Glycine max (soybean) lectin (SAB).

11. The method according to claim 9 or 10 wherein the sample comprises saliva or sputum obtainable from a subject suspected to be infected by the coronavirus.

12. The method according to any one of claims 9-11 wherein the sample comprises material obtained from a surface suspected to be contaminated by the coronavirus and wherein the step b) comprises immersing the wiping means comprising the adsorbed sample to the solution.

13. The method according to any one of claims 9-12 wherein the sample comprises saliva or sputum, and wherein the method comprises exposing the sample to N-acetyl-L-cysteine.

14. Use of a kit in the method according to any one of claims 1-8, the kit comprising
i. a solution comprising
∘ alcohol preferably selected from methanol, ethanol, and isopropanol, more preferably ethanol and isopropanol and mixture thereof, most preferably ethanol,
∘ one or more components selected from sodium dichloroisocyanurate, peroxyacetic acid, sodium dodecyl sulfate, and benzalkonium chloride,
ii. a conjugate comprising
∘ a first lectin,
∘ a detectable label such as colloidal gold and
iii. a probe comprising a detection zone comprising an immobilized second lectin
wherein the first lectin and the second lectin is a O-glycan binding lectin selected from a group consisting of HMA, SAMB SNA-I, Agaricus bisphorus lectin (ABL), Amaranthus caudatus (ACL), Bauhinia forficate (BfL), Bouhinia purpurea (BPA), Grriffonia (Bandeiraea) simplicifolia (GS-IV), soybean agglutinin lectin (SBA)and Glycine max (soybean) lectin (SAB)
and optionally comprising
iv. a solution comprising N-acetyl-L-cysteine.

15. Use of a kit in the method according to any one of claims 9-13, the kit comprising
i. a solution comprising
∘ alcohol preferably selected from methanol, ethanol, and isopropanol, more preferably ethanol and isopropanol and mixture thereof, most preferably ethanol,
∘ one of more components selected from sodium dichloroisocyanurate, peroxyacetic acid, sodium dodecyl sulfate, and benzalkonium chloride,
ii. a lectin array comprising one or more lectins selected from a group consisting of HMA, SAMB SNA-I, Agaricus bisphorus lectin (ABL), Amaranthus caudatus (ACL), Bauhinia forficate (BfL), Bouhinia purpurea (BPA), Grriffonia (Bandeiraea) simplicifolia (GS-IV), soybean agglutinin lectin (SBA), and Glycine max (soybean) lectin (SAB), and optionally, and optionally
iii. a solution comprising N-acetyl-L-cysteine.

## Patentansprüche

1. Verfahren zur Bestimmung des SARS-CoV-2-Coronavirus aus einer Probe, wobei das Verfahren Folgendes umfasst:
a) Bereitstellen
i. einer Lösung (201, 301), welche Folgendes umfasst:
∘ eine oder mehrere Komponenten (202, 302), die zum Neutralisieren des Coronavirus geeignet sind,
∘ eine oder mehrere Komponenten (203, 303), die zum Freilegen von Glykoproteinen des Coronavirus geeignet sind,
ii. ein Konjugat (204, 304, 404), welches Folgendes umfasst:
∘ ein erstes Lektin (206, 306, 406), das zum Binden der Glykoproteine geeignet ist, wobei das Binden selektiv ist,
∘ ein Mittel zur Signalerzeugung (207, 307, 407), und
iii. eine Sonde (205, 305, 405), welche einen Nachweisbereich (205b, 305b, 405b) umfasst, welcher ein immobilisiertes zweites Lektin (209, 309, 409) umfasst, das zum Binden der Glykoproteine geeignet ist, wobei das Binden selektiv ist,
b) Eintauchen der Probe in die Lösung,
c) Aussetzen des Konjugates gegenüber der Lösung, welche die Probe umfasst,
d) Aussetzen des Nachweisbereichs gegenüber der Lösung, welche die Probe und das Konjugat umfasst,
e) vorzugsweise Entfernen von ungebundenem Material aus dem Nachweisbereich,
f) Nachweisen eines Signals, das von dem Mittel zur Signalerzeugung abgeleitet ist, in dem Nachweisbereich, und
g) Bestimmen des SARS-CoV-2-Coronavirus in der Probe basierend auf dem Nachweis, **dadurch gekennzeichnet, dass** das erste Lektin und das zweite Lektin aus O-Glykan-bindenden Lektinen ausgewählt sind.

2. Verfahren nach Anspruch 1, wobei das O-Glykan-bindende Lektin ausgewählt ist aus einer Gruppe bestehend aus Homarus americanus (HMA) -Lektin, Sambucus sieboldiana (SAMB) -Lektin, Sambucus nigra I (SNA-I) -Lektin, Agaricus bisporus (ABL) -Lektin, Amaranthus caudatus (ACL) -Lektin, Bauhinia forficata (BfL) -Lektin, Bauhinia purpurea (BPA) -Lektin, Griffonia (Bandeiraea) simplicifolia (GS-IV) -Lektin, Artocarpus integrifolia (Jacalin) -Lektin, Sojabohnen-Agglutinin (SBA) -Lektin und Glycin max (Sojabohne) (SAB) -Lektin, vorzugsweise ABL und ACL, am bevorzugtesten ABL.

3. Verfahren nach Anspruch 1 oder 2, wobei die Probe Speichel oder Sputum umfasst, der/das von einem Subjekt erhalten werden kann, bei welchem der Verdacht besteht, dass es mit dem Coronavirus infiziert ist.

4. Verfahren nach Anspruch 1 oder 2, wobei die Probe Material umfasst, das von einer Oberfläche erhalten wird, bei welcher der Verdacht besteht, dass sie mit dem Coronavirus kontaminiert ist.

5. Verfahren nach einem der Ansprüche 1 - 4, wobei die Probe auf einem Wischmittel adsorbiert ist und wobei Schritt b) das Eintauchen des Wischmittels, welches die adsorbierte Probe umfasst, in die Lösung umfasst.

6. Verfahren nach einem der Ansprüche 1 - 5, wobei das erste Lektin und das zweite Lektin ähnliche Lektine sind.

7. Verfahren nach einem der Ansprüche 1 - 6, wobei die Probe Speichel oder Sputum umfasst und wobei das Verfahren das Aussetzen der Probe gegenüber N-Acetyl-L-Cystein umfasst.

8. Verfahren nach einem der Ansprüche 1 - 7, wobei die Sonde (405) einen Kontrollbereich (405c) umfasst, der ein immobilisiertes Molekül (411) umfasst, das zum Binden des ersten Lektins geeignet ist, wobei das Binden nicht-selektiv ist, und wobei das Verfahren Folgendes umfasst:
h) Aussetzen des Kontrollbereichs gegenüber der Lösung, welche die Probe und das Konjugat (404) umfasst,
i) vorzugsweise Entfernen von ungebundenem Material aus dem Kontrollbereich,
j) Nachweisen eines Signal, das von dem Mittel zur Signalerzeugung abgeleitet ist, in dem Kontrollbereich, und
k) Bestimmen der Gegenwart oder Abwesenheit des Coronavirus in der Probe, wobei
• das Nachweisen eines Signals in dem Nachweisbereich und in dem Kontrollbereich eine Indikation der Gegenwart des Coronavirus in der Probe ist, und
• die Abwesenheit eines Signals in dem Nachweisbereich und das Nachweisen eines Signals in dem Kontrollbereich eine Indikation der Abwesenheit des Coronavirus in der Probe ist.

9. Verfahren zur Bestimmung des SARS-CoV-2-Coronavirus aus einer Probe, wobei das Verfahren folgende Schritte umfasst:
a) Bereitstellen einer Lösung, welche Folgendes umfasst:
∘ eine oder mehrere Komponenten, die zum Neutralisieren des Coronavirus geeignet sind,
∘ eine oder mehrere Komponenten, die zum Freilegen von Glykoproteinen des Coronavirus geeignet sind,
b) Eintauchen der Probe in die Lösung,
c) Bestimmen von O-Glykanen in der Lösung durch Verwendung eines Lektin-Arrays, welches ein oder mehrere Lektine umfasst, die zum selektiven Binden an O-Glykane von Glykoproteinen geeignet sind, wobei das Binden indikativ für das SARS-CoV-2-Coronavirus in der Probe ist.

10. Verfahren nach Anspruch 9, wobei die O-Glykan-bindenden Lektine des Arrays eines oder mehrere aus einer Gruppe bestehend aus HMA, SAMB, SNA-I, Agaricus bisporus (ABL) -Lektin, Amaranthus caudatus (ACL) -Lektin, Bauhinia forficata (BfL) -Lektin, Bauhinia purpurea (BPA) -Lektin, Griffonia (Bandeiraea) simplicifolia (GS-IV) -Lektin, Artocarpus integrifolia (Jacalin) -Lektin, Sojabohnen-Agglutinin (SBA) -Lektin und Glycin max (Sojabohne) (SAB) -Lektin umfassen.

11. Verfahren nach Anspruch 9 oder 10, wobei die Probe Speichel oder Sputum umfasst, der/das von einem Subjekt erhalten werden kann, bei welchem der Verdacht besteht, dass es mit dem Coronavirus infiziert ist.

12. Verfahren nach einem der Ansprüche 9 - 11, wobei die Probe Material umfasst, das von einer Oberfläche erhalten wird, bei welcher der Verdacht besteht, dass sie mit dem Coronavirus kontaminiert ist und wobei Schritt b) das Eintauchen des Wischmittels, welches die adsorbierte Probe umfasst, in die Lösung umfasst.

13. Verfahren nach einem der Ansprüche 9 - 12, wobei die Probe Speichel oder Sputum umfasst und wobei das Verfahren das Aussetzen der Probe gegenüber N-Acetyl-L-Cystein umfasst.

14. Verwendung eines Kits bei dem Verfahren nach einem der Ansprüche 1-8, wobei das Kit Folgendes umfasst:
i. eine Lösung, welche Folgendes umfasst:
∘ Alkohol, vorzugsweise ausgewählt aus Methanol, Ethanol und Isopropanol, bevorzugter Ethanol und Isopropanol und eine Mischung davon, am bevorzugtesten Ethanol,
∘ eine oder mehrere Komponenten ausgewählt aus Natriumdichlorisocyanurat, Peroxyessigsäure, Natriumdodecylsulfat und Benzalkoniumchlorid,
ii. ein Konjugat, welches Folgendes umfasst:
∘ ein erstes Lektin,
∘ ein nachweisbares Label, wie z. B. kolloidales Gold, und
iii. eine Sonde, welche einen Nachweisbereich umfasst, der ein immobilisiertes zweites Lektin umfasst, wobei das erste Lektin und das zweite Lektin ein O-Glykan-bindendes Lektin ausgewählt aus einer Gruppe bestehend aus HMA, SAMB, SNA-I, Agaricus bisporus (ABL) -Lektin, Amaranthus caudatus (ACL), Bauhinia forficata (BfL), Bauhinia purpurea (BPA), Griffonia (Bandeiraea) simplicifolia (GS-IV), Sojabohnen-Agglutinin (SBA) -Lektin und Glycin max (Sojabohne) (SAB) -Lektin sind,
und wahlweise Folgendes umfasst:
iv. eine Lösung, welche N-Acetyl-L-Cystein umfasst.

15. Verwendung eines Kits bei dem Verfahren nach einem der Ansprüche 9 - 13, wobei das Kit Folgendes umfasst:
i. eine Lösung, welche Folgendes umfasst:
∘ Alkohol, vorzugsweise ausgewählt aus Methanol, Ethanol und Isopropanol, bevorzugter Ethanol und Isopropanol und eine Mischung davon, am bevorzugtesten Ethanol,
∘ eine oder mehrere Komponenten ausgewählt aus Natriumdichlorisocyanurat, Peroxyessigsäure, Natriumdodecylsulfat und Benzalkoniumchlorid,
ii. ein Lektin-Array, welches ein oder mehrere Lektine ausgewählt aus einer Gruppe bestehend aus HMA, SAMB, SNA-I, Agaricus bisporus (ABL) -Lektin, Amaranthus caudatus (ACL), Bauhinia forficata (BfL), Bauhinia purpurea (BPA), Griffonia (Bandeiraea) simplicifolia (GS-IV), Sojabohnen-Agglutinin (SBA) -Lektin und Glycin max (Sojabohne) (SAB) -Lektin umfasst,
und wahlweise
iii. eine Lösung, welche N-Acetyl-L-Cystein umfasst.

## Revendications

1. Procédé destiné à identifier le coronavirus SARS-CoV-2 dans un échantillon, le procédé comprenant :
a) la fourniture
i. d'une solution (201, 301) contenant :
∘ un ou plusieurs composants (202, 302) conçus pour neutraliser le coronavirus,
∘ un ou plusieurs composants (203, 303) conçus pour exposer les glycoprotéines du coronavirus,
ii. d'un conjugué (204, 304, 404) comprenant :
∘ une première lectine (206, 306, 406) conçue pour se lier aux glycoprotéines, où la liaison est sélective,
∘ un moyen de génération d'un signal (207, 307, 407), et
iii. une sonde (205, 305, 405) comprenant une zone de détection (205b, 305b, 405b) comprenant une seconde lectine immobilisée (209, 309, 409) conçue pour se lier aux glycoprotéines, où la liaison est sélective,
b) l'immersion de l'échantillon dans la solution,
c) l'exposition du conjugué à la solution contenant l'échantillon,
d) l'exposition de la zone de détection à la solution contenant l'échantillon et le conjugué,
e) le retrait, de préférence, du matériel non lié de la zone de détection,
f) la détection du signal provenant du moyen de génération d'un signal sur la zone de détection, et
g) l'identification du coronavirus SARS-CoV-2 dans l'échantillon, basée sur la détection, **caractérisée en ce que** la première lectine et la seconde lectine sont sélectionnées parmi des lectines se liant aux O-glycanes.

2. Procédé selon la revendication 1, où la lectine se liant aux O-glycanes est sélectionnée dans le groupe comprenant la lectine *Homarus americanus* (HMA), la lectine *Sambucus sieboldiana* (SAMB), la lectine *Sambucus nigra L.* (SNA-l), la lectine *Agaricus bisporus* (ABL), la lectine *Amaranthus caudatus* (ACL), la lectine *Bauhinia forficata* (BfL), la lectine *Bauhinia purpurea* (BPA), la lectine *Griffonia (Bandeiraea) simplicifoia* (GS-IV), la lectine Artocarpus integrifolia (jacaline), la lectine de soja (SBA) et la lectine de soja glycine max (SAB), de préférence l'ABL et l'ACL, plus préférablement l'ABL.

3. Procédé selon les revendications 1 ou 2, où l'échantillon comprend de la salive ou du crachat provenant d'un sujet suspecté d'être infecté par le coronavirus.

4. Procédé selon les revendications 1 ou 2, où l'échantillon comprend le matériel provenant d'une surface suspectée d'être contaminée par le coronavirus.

5. Procédé selon l'une quelconque des revendications 1 à 4, où l'échantillon est adsorbé sur un dispositif de prélèvement par frottement, et où l'étape b) comprend l'immersion du dispositif de prélèvement par frottement contenant l'échantillon adsorbé dans la solution.

6. Procédé selon l'une quelconque des revendications 1 à 5, où la première lectine et la seconde lectine sont des lectines similaires.

7. Procédé selon l'une quelconque des revendications 1 à 6, où l'échantillon comprend de la salive ou du crachat, et où le procédé comprend l'exposition de l'échantillon à la N-acétyl-L-cystéine.

8. Procédé selon l'une quelconque des revendications 1 à 7, où la sonde (405) comprend une zone de contrôle (405c) comprenant une molécule immobilisée (411) conçue pour se lier à la première lectine, où la liaison est non sélective, et où le procédé comprend :
h) l'exposition de la zone de contrôle à la solution contenant l'échantillon et le conjugué (404),
i) le retrait, de préférence, du matériel non lié de la zone de contrôle,
j) la détection du signal provenant du moyen de génération d'un signal sur la zone de contrôle, et
k) la détermination de la présence ou de l'absence du coronavirus dans l'échantillon, où
• la détection du signal sur la zone de détection et sur la zone de contrôle est une indication de la présence du coronavirus dans l'échantillon, et
• l'absence de signal sur la zone de détection et la détection du signal sur la zone de contrôle est une indication de l'absence du coronavirus dans l'échantillon.

9. Procédé destiné à identifier le coronavirus SARS-CoV-2 dans un échantillon, le procédé comprenant les étapes suivantes :
a) la fourniture d'une solution contenant :
∘ un ou plusieurs composants conçus pour neutraliser le coronavirus,
∘ un ou plusieurs composants conçus pour exposer les glycoprotéines du coronavirus,
b) l'immersion de l'échantillon dans la solution,
c) l'identification d'O-glycanes dans la solution grâce à l'utilisation d'un array de lectines comprenant une ou plusieurs lectines conçues pour se lier de façon sélective aux O-glycanes de glycoprotéines, où la liaison est un indicateur de la présence du coronavirus SARS-CoV-2 dans l'échantillon.

10. Procédé selon la revendication 9, où les lectines de l'array se liant aux O-glycanes consistent en une ou plusieurs lectines provenant d'un groupe constitué de l'HMA, de la SAMB, de la SNA-l, de la lectine *Agaricus bisporus* (ABL), de la lectine *Amaranthus caudatus* (ACL), de la lectine *Bauhinia forficata* (BfL), de la lectine *Bauhinia purpurea* (BPA), de la lectine *Griffonia (Bandeiraea) simplicifoia* (GS-IV), de la lectine *Artocarpus integrifolia* (jacaline), de la lectine de soja (SBA) et de la lectine de soja glycine max (SAB).

11. Procédé selon les revendications 9 ou 10, où l'échantillon comprend de la salive ou du crachat provenant d'un sujet suspecté d'être infecté par le coronavirus.

12. Procédé selon l'une quelconque des revendications 9 à 11, où l'échantillon comprend le matériel provenant d'une surface suspectée d'être contaminée par le coronavirus et où l'étape b) comprend l'immersion du dispositif de prélèvement par frottement contenant l'échantillon adsorbé dans la solution.

13. Procédé selon l'une quelconque des revendications 9 à 12, où l'échantillon comprend de la salive ou du crachat, et où le procédé comprend l'exposition de l'échantillon à la N-acétyl-L-cystéine.

14. Utilisation d'un kit dans le procédé selon l'une quelconque des revendications 1 à 8, le kit comprenant :
i. une solution contenant :
∘ de l'alcool, de préférence sélectionné parmi le méthanol, l'éthanol et l'isopropanol, plus préférablement l'éthanol et l'isopropanol et un mélange de ceux-ci, plus préférablement l'éthanol,
∘ un ou plusieurs composants sélectionnés parmi le dichloroisocyanurate de sodium, l'acide péroxyacétique, le dodécylsulfate de sodium et le chlorure de benzalkonium,
ii. un conjugué comprenant :
∘ une première lectine,
∘ un produit de détection tel que l'or colloïdal, et
iii. une sonde comprenant une zone de détection comprenant une seconde lectine immobilisée
où la première lectine et la seconde lectine sont des lectines se liant aux O-glycanes, sélectionnées dans le groupe comprenant l'HMA, la SAMB, la SNA-l, la lectine *Agaricus bisporus* (ABL), la lectine *Amaranthus caudatus* (ACL), la lectine *Bauhinia forficata* (BfL), la lectine *Bauhinia purpurea* (BPA), la lectine *Griffonia (Bandeiraea) simplicifoia* (GS-IV), la lectine de soja (SBA) et la lectine de soja glycine max (SAB) et comprenant facultativement
iv. une solution contenant de la N-acétyl-L-cystéine.

15. Utilisation d'un kit dans le procédé selon l'une quelconque des revendications 9 à 13, le kit comprenant :
i. une solution contenant :
∘ de l'alcool, de préférence sélectionné parmi le méthanol, l'éthanol et l'isopropanol, plus préférablement l'éthanol et l'isopropanol et un mélange de ceux-ci, plus préférablement l'éthanol,
∘ un ou plusieurs composants sélectionnés parmi le dichloroisocyanurate de sodium, l'acide péroxyacétique, le dodécylsulfate de sodium et le chlorure de benzalkonium,
ii. un array de lectines comprenant une ou plusieurs lectines sélectionnées dans le groupe comprenant l'HMA, la SAMB, la SNA-l, la lectine *Agaricus bisporus* (ABL), la lectine *Amaranthus caudatus* (ACL), la lectine *Bauhinia forficata* (BfL), la lectine *Bauhinia purpurea* (BPA), la lectine *Griffonia (Bandeiraea) simplicifoia* (GS-IV), la lectine de soja (SBA) et la lectine de soja glycine max (SAB), et facultativement
iii. une solution contenant de la N-acétyl-L-cystéine.
